# EUROPEAN PATENT SPECIFICATION

(11) **EP 2 525 753 B1**
(45) Date of publication and mention of the grant of the patent: **25.11.2020**
(21) Application number: 10821525.2
(22) Date of filing: 05.10.2010
(51) Int. Cl.: A61F 11/10, F16K 15/20, F16K 17/04, H04R 25/02, H04R 25/00, A61F 11/08

(54) **PRESSURE REGULATION MECHANISM FOR INFLATABLE IN-EAR DEVICE**
DRUCKREGULIERUNGSMECHANISMUS FÜR AUFBLASBARE INNEROHRVORRICHTUNGEN
MÉCANISME DE RÉGULATION DE PRESSION POUR DISPOSITIF INTRA-AURICULAIRE GONFLABLE

(30) Priority: 05.10.2009 US 272534 P
(43) Date of publication of application: 28.11.2012
(73) Proprietor: Sonomax Technologies Inc., Montréal, Québec H4P 2E2 (CA)
(72) Inventor: TURCOT, Michael C., Montreal, Quebec H4A 2V6 (CA); VOIX, Jérémie, Montreal, Quebec H2R 2B6 (CA)
(74) Representative: Patentanwaltskanzlei Matschnig & Forsthuber OG
(86) International application number: PCT/CA2010/001602
(87) International publication number: WO 2011/041900

(56) References cited:
- WO-A1-2008/070986
- WO-A1-2009/026532
- WO-A2-2009/077902
- WO-A2-2009/077902
- CN-A- 101 098 566
- DE-A1- 2 737 252
- US-A- 3 110 356
- US-A- 4 913 165
- US-A- 5 006 055
- US-A- 5 006 055
- US-A1- 2002 114 479

## Description

### FIELD OF THE INVENTION

The present invention concerns an inflatable in-ear device and in particular has reference to a component thereof for modulating and controlling the inflation pressure during inflation.

In-ear devices include intra-aural hearing protectors (earplugs), earphones, hearing-aid devices and the like and the invention relates to custom-fitting in-ear devices that are formed *in situ* to conform morphologically to the inside of the ear canal and the *cavum concha* of the individual.

### BACKGROUND OF THE INVENTION

The present inventors have proposed hearing protection devices which offer custom-fitting to the ear of the individual and are exemplified and claimed in US Patent Nos. 6,339,648 and 6,754,357 to McIntosh et al. Principally, these devices comprise a core member around which is arranged a sheath in a deflated state, a permanent setting compound being injected into the cavity defined between the core member and the sheath to bring the device into morphological conformity to the contours of the ear canal. In this way, the in-ear device is produced in customized fashion thus enhancing the performance thereof and indeed the comfort of the individual.

The sheath is typically produced from a silicon material having hydrodynamic properties enabling a quasi-isobaric inflation process which maintains a substantially constant pressure (P1) during inflation without external constraints (similarly to an inflatable balloon), which could typically be in the order of about 0.8 psig (about 5,5 kPa relative pressure). However, when constrained within the ear canal, the internal pressure rises and causes the in-ear device to overinflate and may injure or cause discomfort to the wearer of the device (at a pressure above a comfort-limit of ear canal pressure level P2) when a settable compound is injected between the core and the sheath through an injection channel, which is in the order of about 2.0 psig (about 14 kPa relative pressure). US patent No. 6,687,377 and in US patent application No. 2005-0123146A1 tentatively try to solve that problem of limiting the maximum pressure level by assessing *in situ* the acoustic attenuation of the in-ear device during the inflation mode.

Document DE 2737252 A1 to Koken Co discloses a plug-type hearing device having an envelope that expands to closely contact with the wall surface of the auditory meatus.

Also, US 5,006,055 discloses an apparatus for manufacturing an otoplastic directly in the ear of a hearing-impaired person.

Accordingly, there is a need for an improved mechanism capable of regulating the pressure during the inflation mode of an inflatable in-ear device.

### SUMMARY OF THE INVENTION

A general object of the present invention is thus to provide a mechanism capable of regulating the pressure during the inflation mode of an inflatable in-ear device that solves the above-mentioned problems. The invention is described in the attached claim 1.

An advantage of the present invention is that the pressure regulating mechanism has a maximum pressure limit set at a value that ensures the in-ear device morphologically conforms to the inside of the ear canal and the *cavum concha* of the individual without discomfort.

Another advantage of the present invention is that the pressure regulating mechanism ensures that the quantity of compound delivered for the adaptation of the in-ear device to the geometry of a human ear canal is sufficient to allow proper expansion to assume the contour of the ear canal and the *cavum concha* of the individual.

A further advantage of the present invention is that a facility is provided for limiting the amount of settable compound delivered for inflation of the sheath for the purpose of conforming morphologically to the ear canal.

Yet another advantage of the present invention is that the pressure regulating mechanism includes a setting pressure mechanism that enables the user to vary the maximum pressure limit to ensure a better noise protection from the inflatable in-ear device wherever required, even though some discomfort could be tolerated by the user for short period of times.

According to the present invention, there is provided an inflatable in-ear device comprising a core member of generally similar form to the contour of the ear canal of an individual, a sound bore being defined within and through the core member from a region exterior to the ear canal to a relatively inner region within the ear canal, a platform to which the core member is attached exteriorly of the ear canal, an inflatable open-ended sheath having a closed end secured to the relatively inner region of the core member, the sheath assuming a first condition and being foldable into an inflatable condition in close adjacency to the core member prior to inflation, the core member and the platform having communicating injection channels for injecting a settable compound into a space defined between the core member and the sheath in its inflatable condition, an outflow channel communicating with the space allowing discharge of any excess settable compound, and a pressure regulating mechanism associated with the outflow channel.

Conveniently, the pressure regulating mechanism may function also as a mechanism for limiting the amount of settable compound injected into the said space.

According to the invention, the main body of the pressure regulating mechanism is in the form of a relief or unloading valve enabling the modulation of pressure and control of the amount of material within the void between the sheath and the core member during inflation and prior to setting.

Other objects and advantages of the present invention will become apparent from a careful reading of the following description.

### BRIEF DESCRIPTION OF THE DRAWINGS

Further aspects and advantages of the present invention will become better understood with reference to the description in association with the following figures, in which similar references used in different figures denote similar components, wherein:
**Figure 1** is a partial schematic cross-sectional view of an inflatable in-ear device with a pressure regulating mechanism not falling under the scope of the claims;
**Figure 2** is a view similar to Figure 1 showing a second example of pressure regulating mechanism not falling under the scope of the claims;
**Figure 3** is a view similar to Figure 1 showing a pressure regulating mechanism in accordance with the present invention; and
**Figure 4** is a view similar to Figure 1 showing another example of pressure regulating mechanism not falling under the scope of the claims.

### DETAILED DESCRIPTION OF THE PREFERRED EMBODIMENTS

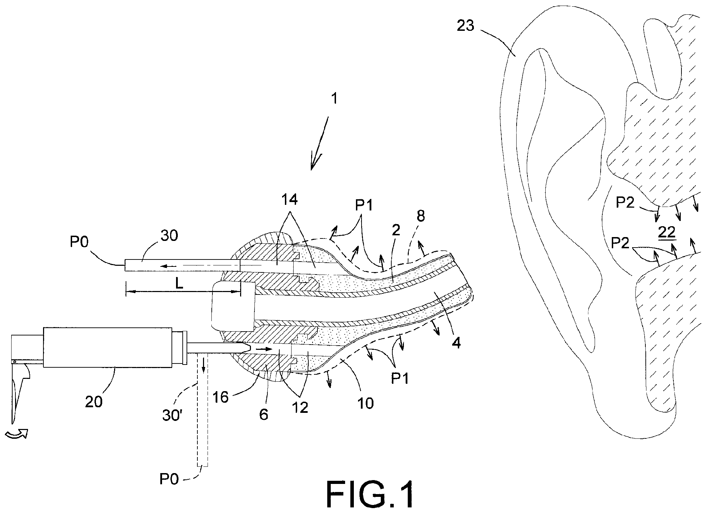

Referring to the drawings there is illustrated in Figures 1, 2, 3 and 4 an inflatable in-ear device 1 comprising a core member 2 with a sound bore 4 extending therethrough and into a platform 6 to which the member 2 is attached. A sheath 8 of flexible material surrounds the core member 2 (shown non-uniformly spaced from the core member 2, as if separated therefrom by a compound material, for the purpose of clarity) and provides a space 10 for the injection of a settable compound (not shown but inside body of 20), such as a silicone compound or the like. The core member 2 and the platform 6 are both provided with injection and outflow channels 12, 14 for respectively allowing the ingress and the egress of the compound into and out of the space 10. The platform 6 is provided with an end capping 16 which is so formed as to allow access to the inflow and outflow channels. An injection syringe 20 is shown in the figures as the means of injecting the compound in use into the space 10, but it will be understood that other means may be employed. The device 1 is shown poised for insertion into the ear canal 22 of an individual whose ear 23 is illustrated.

Generally, the inflatable in-ear device 1 is duly inserted into the ear canal 22 and the settable compound (not shown) is injected by use of the syringe 20 through the injection channel 12 to inflate the sheath 8 and fill the space 10, between the sheath 8 and the core member 2, thereby causing the sheath 8 to conform morphologically to the contour of the ear canal 22. When the pressure of the compound reaches the appropriate predetermined pressure level P0, the compound will discharge through the outflow channel 14 via a pressure regulator mechanism.

In the first example of Figure 1 the pressure regulation mechanism has a main body in the form of a length 'L' of tubing 30 which is typically sufficient to receive to whole amount of settable compound, and therefore any possible excess thereof. The predetermined pressure P0 is maintained substantially constant during the flow of the settable compound inside the tubing 30, since the pressure P0 is the pressure required to displace the compound therealong in a so-called, in fluid dynamics, creeping flow, and essentially depends on physical characteristics such as the viscosity of the compound, the nature of the material and the internal diameter of the tubing. The predetermined pressure level P0 is obviously larger than the pressure level P1 required to inflate the sheath 8 (level P1 depends essentially on the material of the sheath and its thickness) and smaller than the pressure level P2 at which an individual may start feeling discomfort into the ear canal 22, as mentioned hereinabove, to allow the inflation of the sheath while preventing overinflation thereof.

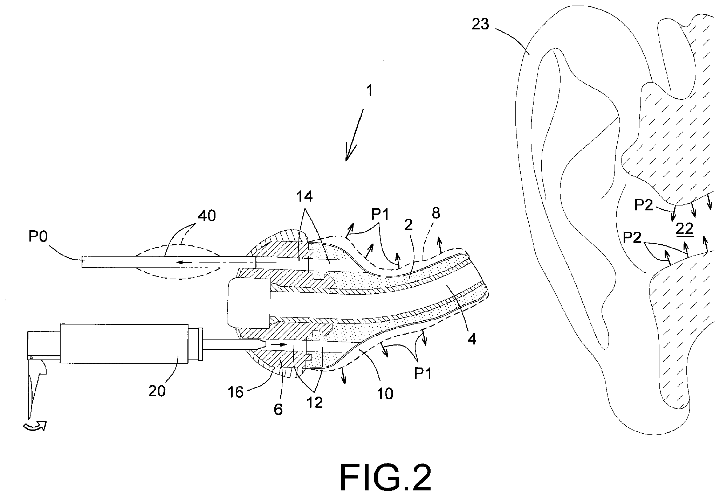

In the second example shown in Figure 2 the main body of the pressure regulating mechanism comprises an inflatable bladder 40 for receiving excess settable compound. The bladder 40, made out of any suitable material, is operable as a second external sheath having quasi-isobaric inflation pressure level P0 larger than P1 but lower than P2, the bladder inflating thereby to act as a valve to relieve pressure and to allow discharge of excess compound.

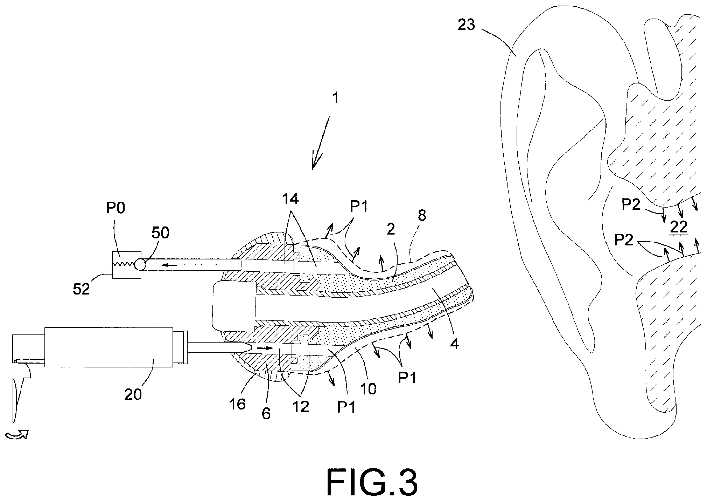

The pressure regulating mechanism Z according to the invention is shown in Figure 3 and essentially comprises a relief/unloader valve 50 mounted on a reservoir 52. When the pressure reaches the predetermined level P0, the valve 50 will open and excess compound will be discharged into the reservoir 52, thus ensuring that the pressure in the sheath 8 does not exceed a level P2 acceptable to the individual and that the amount of compound is not excessive for the comfort of the individual.

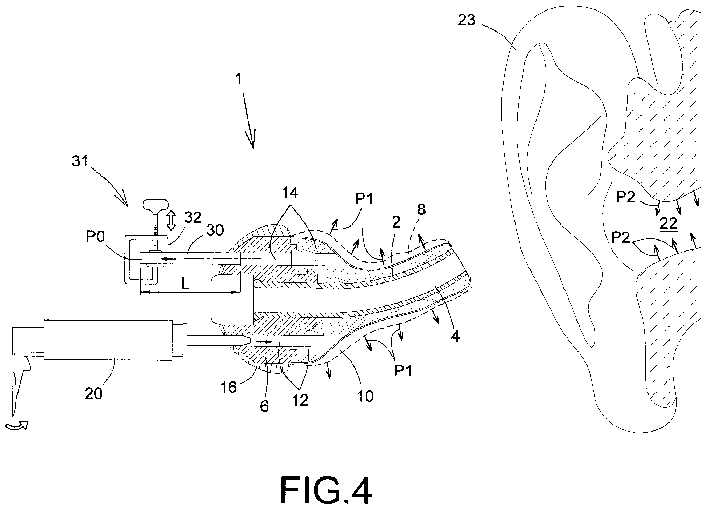

Another example of pressure regulating mechanism is shown in Figure 4 and essentially comprises an external setting pressure applicator 31 which in this example is in the form of a screw clamp 32. The applicator 31 enables the user to vary the maximum pressure limit to ensure a better noise protection from the inflatable in-ear device wherever required by restricting the passage within the tubing 30 to modulate pressure and flow therethrough. For example, such variation is useful in applications such as in shooting training facilities for security forces, or in the military environment among others, even though some discomfort could be tolerated by the user for short periods of times.

Although the four pressure regulating mechanisms in Figures 1 through 4 are shown located substantially downstream of the space 10 in communication with the outflow channels 14, it would be obvious to one skilled in the art that it could be located upstream of the space 10 (and downstream of the syringe 20), as illustrated in dotted lines in Figure 1 for the length of tubing 30' with an equivalent outflow channel.

Although the present invention has been described with a certain degree of particularity, it is to be understood that the disclosure has been made by way of example only and that the present invention is not limited to the features of the embodiments described and illustrated herein, but includes all variations and modifications within the scope of the invention as hereinafter claimed.

## Claims

1. A system comprising an inflatable in-ear device (1) and a pressure regulating mechanism configured to regulate a pressure of a settable compound being injected into the inflatable in-ear device (1), said in-ear device (1) including a core member (2) adapted to fit inside of the ear canal (22) of an individual, a sound bore (4) being defined within and through the core member (2) from a region exterior to the ear canal to a relatively inner region within the ear canal, a platform (6) to which the core member (2) is attached exteriorly of the ear canal, an inflatable open-ended sheath (8) having a closed end secured to the relatively inner region of the core member (2), the sheath (8) assuming a first condition in close adjacency to the core member (2) prior to inflation and being foldable into an inflatable condition under an inflation pressure level (P1) determined by the sheath material and thickness, the core member (2) and the platform (6) having communicating injection channels (12) and outflow channels (14) for ingress and egress of a settable compound into and out of a space (10) defined between the core member (2) and the sheath (8) in its inflatable condition, and said pressure regulating mechanism being **characterized by** a main body (30) connecting to the outflow channel (14) communicating with the space (10), said main body including a relief valve (50) connected to the outflow channel (14) mounted on a reservoir (52), the relief valve (50) being adapted to open when the pressure of the settable compound reaches a predetermined pressure level (P0) and to allow all excess of settable compound to be discharged into the reservoir (52) during inflation of the sheath (8) while maintaining the pressure of the settable compound at the predetermined pressure level (P0), the predetermined pressure level (P0) being larger than the inflation pressure level (P1) to ensure inflation of the sheath (8) and smaller than a comfort-limit of ear canal pressure level (P2).

## Patentansprüche

1. System aufweisend eine aufblasbare, im Ohr tragbare Vorrichtung (1) und einem Druckregelmechanismus, der zum Steuern des Drucks einer härtbaren Verbindung, die in die aufblasbare, im Ohr tragbare Vorrichtung (1) injiziert wird, eingerichtet ist, wobei die im Ohr tragbare Vorrichtung (1) umfasst: ein Kernelement (2), das in den Gehörgang (22) eines Individuums passend ausgelegt ist, wobei eine Schallbohrung (4) in dem Kernelement (2) und dieses durchlaufend von einem Bereich außerhalb des Gehörgangs zu einem relativ inneren Bereich innerhalb des Gehörgangs definiert ist, eine Plattform (6), an der das Kernelement (2) außerhalb des Gehörgangs angebracht ist, eine aufblasbare Hülle (8) mit offenem Ende und mit einem geschlossenen Ende, das an dem relativ inneren Bereich des Kernelements (2) befestigt ist, wobei die Hülle (8) einen ersten Zustand in enger Nähe zu dem Kernelement (2) vor dem Aufblasen einnimmt und bei einem durch das Material und die Dicke der Hülle bestimmten Aufblasdruck-Niveau (P1) in einen aufblasbaren Zustand faltbar ist, wobei das Kernelement (2) und die Plattform (6) miteinander in Verbindung stehende Injektionskanäle (12) und Abflusskanäle (14) für den Eintritt und den Austritt einer härtbaren Verbindung in einen zwischen dem Kernelement (2) und der Hülle (8) im aufblasbaren Zustand definierten Raum (10) und aus diesem heraus aufweisen, und Druckregelmechanismus **gekennzeichnet durch** einen Hauptkörper (30), der mit dem mit dem Raum (10) kommunizierenden Abflusskanal (14) verbunden ist, wobei der Hauptkörper ein mit dem Abflusskanal (14) verbundenes Überdruckventil (50) aufweist, das auf einem Reservoir (52) montiert ist, wobei das Überdruckventil (50) dazu eingerichtet ist, sich zu öffnen, wenn der Druck der härtbaren Verbindung ein vorbestimmtes Druckniveau (P0) erreicht, und zu ermöglichen, dass während des Aufblasens der Hülle (8) sämtlicher Überschuss der härtbaren Verbindung in das Reservoir (52) abgelassen wird, während der Druck der härtbaren Verbindung auf dem vorbestimmten Druckniveau (P0) gehalten wird, wobei das vorbestimmte Druckniveau (P0) größer ist als das Aufblasdruck-Niveau (P1) ist, um das Aufblasen der Hülle (8) sicherzustellen, und kleiner ist als eine Komfortgrenze des Druckniveaus (P2) im Gehörgang.

## Revendications

1. Système comportant un dispositif intra-auriculaire gonflable (1) et un mécanisme de régulation de pression configuré pour réguler une pression d'un composé durcissable qui est injecté dans le dispositif intra-auriculaire gonflable (1), ledit dispositif intra-auriculaire (1) comprenant un élément principal (2) adapté pour s'ajuster à l'intérieur du conduit auditif (22) d'un individu, un perçage sonore (4) étant défini à l'intérieur du et à travers l'élément principal (2) d'une région extérieure au conduit auditif jusqu'à une région relativement intérieure dans le conduit auditif, une plate-forme (6) à laquelle l'élément principal (2) est fixé à l'extérieur du conduit auditif, une gaine gonflable à extrémité ouverte (8) ayant une extrémité fermée fixée à la région relativement intérieure de l'élément principal (2), la gaine (8) prenant une première condition à proximité immédiate de l'élément principal (2) avant être gonflée et étant pliable dans une condition gonflable sous un niveau de pression de gonflage (P1) déterminé par le matériau et l'épaisseur de la gaine, l'élément principal (2) et la plate-forme (6) ayant des canaux d'injection (12) et des canaux de sortie (14) communicants pour l'entrée et la sortie d'un composé durcissable dans et hors d'un espace (10) défini entre l'élément principal (2) et la gaine (8) dans sa condition gonflable, et ledit mécanisme de régulation de pression étant **caractérisé par** un corps principal (30) relié au canal de sortie (14) communiquant avec l'espace (10), ledit corps principal comprenant une soupape de surpression (50) reliée au canal de sortie (14) montée sur un réservoir (52), la soupape de décharge (50) étant adaptée pour s'ouvrir lorsque la pression du composé durcissable atteint un niveau de pression prédéterminé (P0) et pour permettre à tout excès de composé durcissable d'être déchargé dans le réservoir (52) pendant le gonflage de la gaine (8) tout en maintenant la pression du composé durcissable au niveau de pression prédéterminé (P0), le niveau de pression prédéterminé (P0) étant supérieur au niveau de pression de gonflage (P1) pour assurer le gonflage de la gaine (8) et inférieur à une limite de confort du niveau de pression du conduit auditif (P2).
